# EUROPEAN PATENT SPECIFICATION

(11) **EP 1 237 588 B1**
(45) Date of publication and mention of the grant of the patent: **01.12.2004**
(21) Application number: 00986404.2
(22) Date of filing: 14.12.2000
(51) Int. Cl.: A61L 31/12, A61L 27/48

(54) **SURGICAL PROSTHESIS**
CHIRURGISCHE PROTHESE
PROTHESE CHIRURGICALE

(30) Priority: 17.12.1999 US 466697
(43) Date of publication of application: 11.09.2002
(73) Proprietor: Genzyme Corporation, Cambridge, MA 02142 (US)
(72) Inventor: GREENAWALT, Keith, E., Milton, MA 02186 (US); BUTLER, Timothy, J., Malden, MA 01864 (US)
(74) Representative: Wallace, Sheila Jane
(86) International application number: PCT/US2000/033971
(87) International publication number: WO 2001/043789

(56) References cited:
- EP-A- 0 705 878
- WO-A-00/16822
- US-A- 5 632 979
- GREENAWALT KEITH E ET AL: "Evaluation of Sepramesh biosurgical composite in a rabbit hernia repair model." JOURNAL OF SURGICAL RESEARCH, [Online] vol. 94, no. 2, 1 November 2000 (2000-11-01), pages 92-98, XP002166286 Retrieved from the Internet: <URL:http://a96.g.akamaitech.net/n/96/1861 /974908308000/extra.idealibrary.com/produc tion/jsre/2000/94/2/jsre.2000.6020/6020a.p df?sessionID=D2J5VJIAAADFYCQAABYQAAA> [retrieved on 2001-04-27]

## Description

### BACKGROUND OF THE INVENTION

An unwanted opening in a body cavity, such as an incisional hernia, is often repaired using a prosthetic mesh (e.g., a polypropylene mesh) to line the inner surface of the body cavity wall at the opening. Viscera in the body cavity display a strong tendency to adhere to exposed mesh during the healing process. This tendency frequently results in significant postoperative complications.

### SUMMARY OF THE INVENTION

In general, the invention provides a laminated surgical prosthesis and methods of making and using the prosthesis. The prosthesis includes a non-bioabsorbable layer having an outer wall-facing surface and an inner bioabsorbable layer-facing surface, an adhesive, and a bioabsorbable layer containing hyaluronic acid (HA) and/or carboxymethyl cellulose (CMC). The HA and CMC (HA/CMC) can be chemically modified e.g., as described in U.S. Patent No. 5,017,229.

The non-bioabsorbable layer can be a mesh of polypropylene or poly(ethylene terephthalate).

The bioabsorbable layer has an outer viscera-facing surface and an inner non-bioabsorbable layer-facing surface which is attached to the bioabsorbable layer-facing surface by the adhesive. In addition, the inner non-bioabsorbable layer-facing surface of the bioabsorbable layer is preferably porous to facilitate binding with the inner bioabsorbable layer-facing surface of the non-bioabsorbable layer. The pores can be about 10-500 µm in diameter, (e.g., 30-300 or 40-100 µm in diameter) and can traverse the bioabsorbable layer from the outer viscera-facing surface to the inner non-bioabsorbable layer-facing surface. The amount of HA and CMC in the bioabsorbable layer can vary. In one example, the ratio of the amount of HA to the amount of CMC is between 1:0.01 and 0.01:1. Exemplary HA:CMC ratios are 1:2, 1:1 and 2:1.

The various layers of the prosthesis can have selected densities as follows. The non-bioabsorbable layer may have a density of about 68 to 102 g/m² (e.g. 85 g/m²) (6.3 to 9.5 g/ft² (e.g., 7.9 g/ft²)). The bioabsorbable layer may have a density of about 22 to 48 g/m² (e.g. 32 g/m²) (2.0 to 4.5 g/ft² (e.g., 3.0 g/ft²)). The adhesive may have a density of about 29 to 44 g/m² (e.g. 37 g/m²) (2.7 to 4.1 g/ft² (e.g., 3.4 g/ft²)).

The adhesive is preferably bioabsorbable and may contain polyglycolic acid, polylactic acid, polycaprolactone, polydioxanone, polyestercarbonate, polyhydroxyalkonate, or copolymers thereof. For example, the adhesive can be a 1:1 copolymer of polyglycolic acid and polylactic acid.

The invention also provides for method of repairing an opening in a wall enclosing a body cavity by positioning the surgical prosthesis of the invention over the opening and in contact with an inner surface of the wall, then closing the opening. The method of repairing an opening in a wall may also preferably include securing the prosthesis to the wall, e.g., by suturing.

In addition, the invention features a method of producing a surgical prosthesis by applying an adhesive to a surface of a non-bioabsorbable sheet, and adhering a bioabsorbable composition to the surface of the non-bioabsorbable sheet subsequent to the application of the adhesive to the surface of the non-bioabsorbable sheet. The composition preferably contains hyaluronic acid and/or carboxymethyl cellulose. The adhering step may include placing the bioabsorbable composition onto the surface of the non-bioabsorbable sheet and heat-pressing the composition in a process such as lamination.

As used herein, "non-bioabsorbable layer" means a layer that contains components that are not readily absorbed, degraded, or otherwise decomposed when present in a body cavity (e.g., the human peritoneal cavity).

As used herein, "bioabsorbable layer" means a layer containing components that can be degraded or absorbed at some time after implantation of the prosthesis, such as within weeks or months following implantation. The bioabsorbable products are preferably eliminated from the body or metabolized.

As used herein, "hyaluronic acid" and "carboxymethyl cellulose" means those compounds and the chemical derivatives thereof, e.g., as described in U.S. Patent No. 5,017,229.

As used herein, "heat-pressing" means a process that involves pressing at least two materials into contact with each other while heat is applied to at least one of the materials. The invention also provides a surgical prosthesis as described above for use in repairing an opening in a wall enclosing a body cavity of a human patient.

The invention can also be used in a method of repairing an opening in a wall enclosing a body cavity of a human patient, the method comprising (i) providing a surgical prosthesis formed of a non-bioabsorbable layer having a wall-facing surface and a bloabsorbable layer-facing surface, an adhesive, and a bioabsorbable layer comprising a material selected from the group consisting of hyaluronic acid and carboxymethyl cellulose, the bioabsorbable layer having a viscera-facing surface and a non-bioabsorbable layer-facing surface attached to the bioabsorbable layer facing surface by the adhesive, wherein the bioabsorbable layer contains pores in the non-bioabsorbable layer-facing surface; (ii) positioning the surgical prosthesis over the opening of the patent, in contact with an inner surface of the wall; and (iii) closing the opening.

In this method, the bioabsorbable layer preferably comprises hyaluronic acid and carboxymethyl cellulose, with the ratio of the total amount of hyaluronic acid to the total amount of carboxymethyl cellulose in the bioabsorbable layer preferably being 2:1. Preferably the pores of the bioabsorbable layer traverse the bioabsorbable layer from the viscera-facing surface to the non-bioabsorbable layer-facing surface.

In another embodiment of the invention, there is provided a method of producing a prosthesis, the method comprising providing a non-bioabsorbable sheet; applying an adhesive to a surface of the non-bioabsorbable sheet; and adhering a bioabsorbable composition to the surface subsequent to application of the adhesive to the surface, the bioabsorbable composition comprising a material selected from the group consisting of hyaluronic acid and carboxymethyl cellulose. The adhering step preferably comprises placing the bioabsorbable layer onto the surface and heat-pressing the composition to the surface.

Also within the scope of the invention is a surgical prosthesis comprising a non-bioabsorbable layer having an outer wall-facing surface and an inner bioabsorbable layer-facing surface; an adhesive; and a bioabsorbable layer having an outer viscera-facing surface and an inner non-bioabsorbable layer-facing surface attached to the bioabsorbable layer-facing surface of the non-bioabsorbable layer by the adhesive, wherein the bioabsorbable layer contains pores in the non-bioabsorbable layer-facing surface.

Alternatively, the surgical prosthesis may comprise a non-bioabsorbable layer having an outer wall-facing surface and an inner bioabsorbable layer-facing surface; an adhesive; and a bioabsorbable layer having an outer viscera-facing surface and an inner non-bioabsorbable layer-facing surface attached to the bioabsorbable layer-facing surface of the non-bioabsorbable layer by the adhesive, wherein the non-bioabsorbable layer has a density of about 68 to 102 g/m² (e.g. 85 g/m²) (6.3 to 9.5 g/ft² (e.g., 7.9 g/ft²)), the bioabsorbable layer has a density of about 22 to 48 g/m² (e.g. 32 g/m²) (2.0 to 4.5 g/ft²) and the adhesive has a density of about 29 to 44 g/m² (e.g. 37 g/m²) (2.7 to 4.1 g/ft²).

Other features and advantages of the invention will be apparent from the following detailed description, figures, and claims.

### BRIEF DESCRIPTION OF THE DRAWING

Fig. 1 is a perspective view of a prosthesis of the invention, with the bioabsorbable (HA/CMC) layer and adhesive partially separated from the non-bioabsorbable layer.

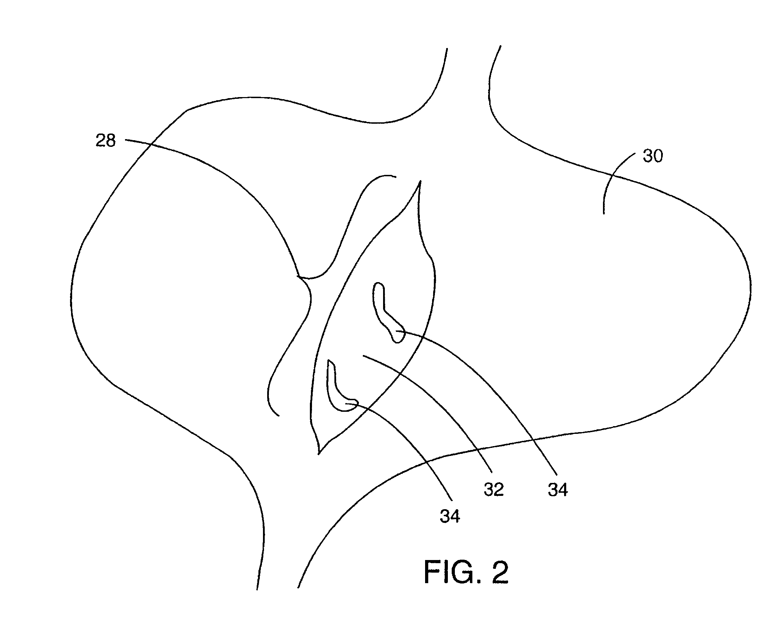

Fig. 2 is a plan view of an opening in a wall of a body cavity before repair.

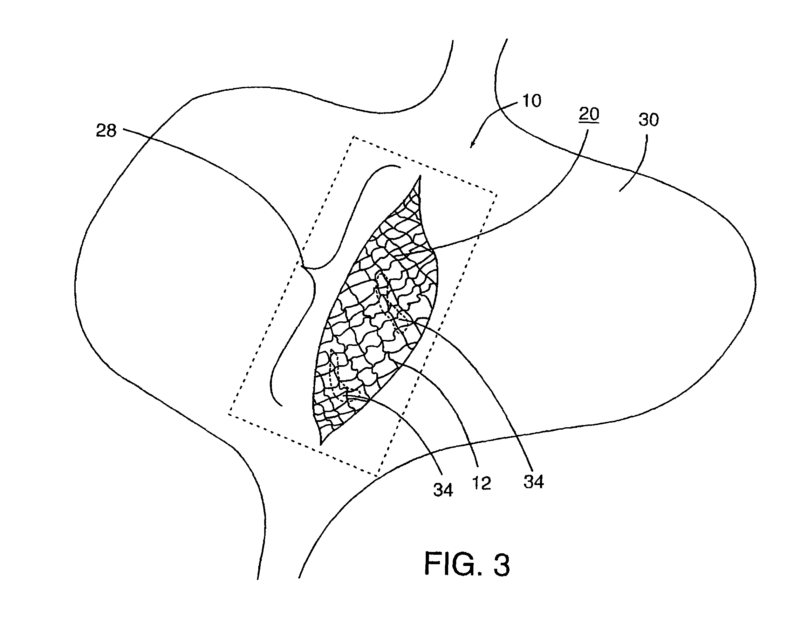

Fig. 3 is a plan view of the opening in Fig. 2 with the prosthesis shown in Fig. 1 properly positioned for repair.

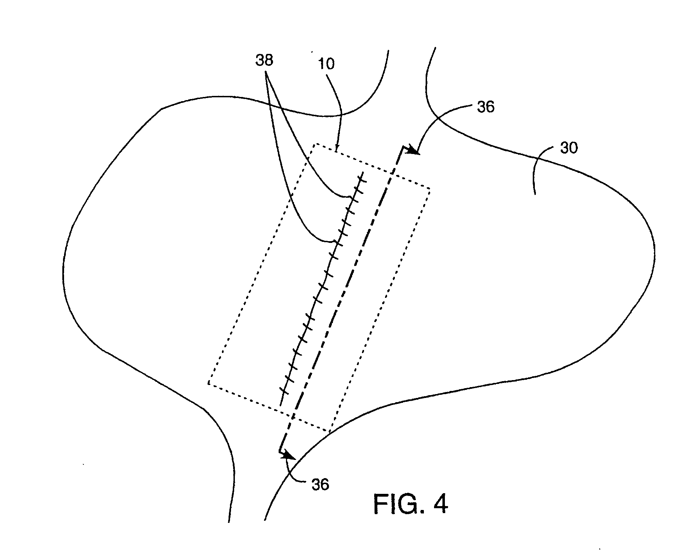

Fig. 4 is a plan view of the opening and prosthesis in Fig. 3, the opening now being closed by sutures.

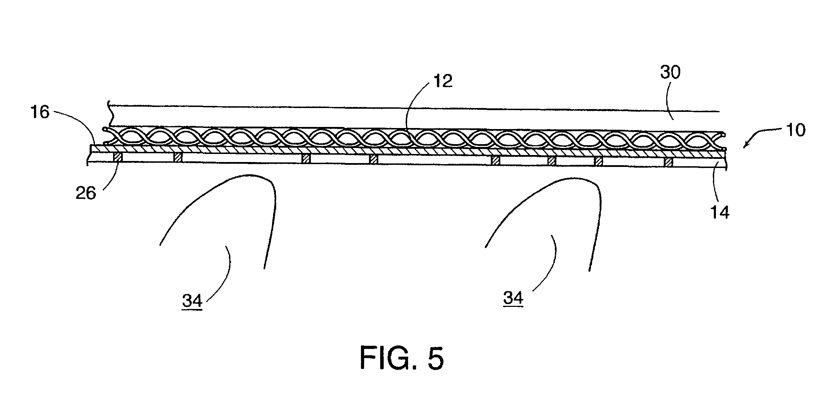

Fig. 5 is a cross sectional view of the repaired wall taken at line 36 shown in Fig. 4.

### DETAILED DESCRIPTION

In general, the invention relates to a surgical prosthesis having at least three layers. A first non-bioabsorbable layer, is made of a material such as polypropylene and can have a thickness of about 400 to 650 microns or a density of about 68 to 102 g/m² (e.g. 85 g/m²) (6.3 to 9.5 g/ft² (e.g., 7.9 g/ft²)). A second layer that is bioabsorbable is made from a material such as hyaluronic acid, carboxymethyl cellulose, or mixtures thereof and can have a thickness of about 150 to 300 microns or a density of about 22 to 48 g/m² (e.g. 32 g/m²) (2.0 to 4.5 g/ft² (e.g., 3.0 g/ft²)). The first and second layers are affixed to each other by a third layer, i.e., an adhesive, formed of a material such as a copolymer of polylactic acid and polyglycolic acid. The third layer can have a thickness of about 25 to 200 microns or a density of about 29 to 44 g/m² (e.g. 37 g/m²) (2.7 to 4.1 g/ft² (e.g., 3.4 g/ft²)).

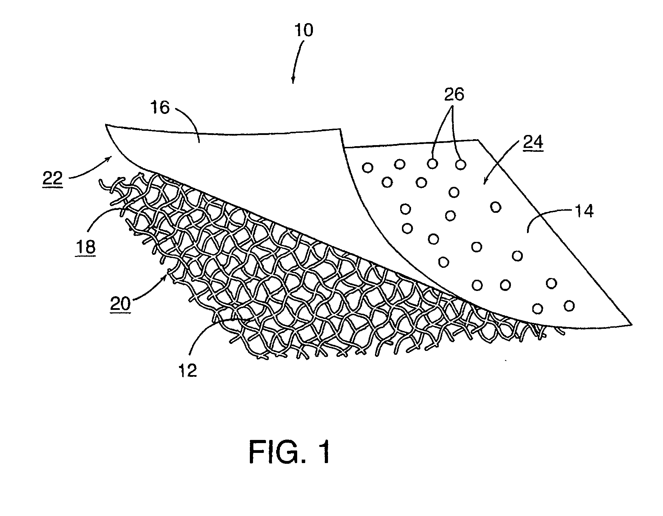

Referring to Fig. 1, a prosthesis 10 includes a non-bioabsorbable mesh 12 made of a polymer such as polypropylene or poly(ethylene terephthalate). The mesh may include woven strands, a woven mesh of fibers, a preformed square pattern, or similar configurations. Mesh 12 includes a wall-facing surface 20 and a bioabsorbable layer-facing surface 18. Bioabsorbable layer 14 is preferably formed of HA and CMC and has non-bioabsorbable layer-facing surface 22 affixed to bioabsorbable layer-facing surface 18 of mesh 12 by an adhesive 16. The adhesive 16 may be, for example, a 1:1 copolymer of polyglycolic acid and polylactic acid. The bioabsorbable layer 14 also includes a viscera-facing surface 24. The bioabsorbable layer 14 contains pores 26 that are adjacent to the non-bioabsorbable layer-facing surface and more preferably traverse the layer from viscera-facing surface 24 to non-bioabsorbable layer-facing surface 22. Although the pores traverse the layer in this particular embodiment, it is to be understood that in other embodiments (not shown), the pores may not traverse the entire bioabsorbable layer. During manufacturing, the adhesive 16 preferably infiltrates the pores 26 and facilitates the binding of bioabsorbable layer 14 to the mesh 12.

In some embodiments the wall-facing surface 20 and the viscera facing surface 24 of the bioabsorbable layer 14 differ in coloration, visible surface markings or a tactile feature. This facilitates identification of the respective surfaces and proper orientation of the prosthesis 10 by the surgeon.

As shown in the drawings, the prosthesis 10 may be used to repair an opening 28 in a wall 30 of a body cavity 32 that contains a viscera surface 34 (e.g., bowel, omentum, etc.). To repair the opening 28, the prosthesis 10 is inserted into body cavity 32 at opening 28. The prosthesis 10 is positioned so the wall-facing surface 20 of mesh 12 covers the opening 28 of the body cavity 32 with the wall-facing surface 20 facing the wall 30 of the body cavity, and the bioabsorbable layer-facing surface 18 faces the viscera 34. The bioabsorbable layer 14 covers the bioabsorbable layer-facing surface 18 and protects the viscera 34 from adhering to the mesh 12 during healing. The repair of the opening 28 in body cavity 32 is then completed by closing the opening 28 with sutures 38.

In time, the bioabsorbable layer 14 is absorbed by the body, leaving bioabsorbable layer-facing surface 18 of the mesh 12 directly facing the viscera 34. However, by the time bioabsorbable layer 14 is absorbed, the opening 28 has healed to an extent where the tendency to form adhesions between the wall 30 of the body cavity and the mesh 12 has abated. This abatement is the result of the reestablishment of a mesothelial lining on the inside surface of wall 30, over healed opening 28. During the healing process, the mesh 12 attaches to the wall of the body cavity.

Based on the above description and the examples described below, one skilled in the art can utilize the present invention to its fullest extent. The following examples are illustrative of how one skilled in the art may make and use the prosthesis of the invention and are not to be construed as limiting the remainder of the disclosure in any way. Any publications cited in this disclosure are hereby incorporated by reference.

### Example 1

A 2.54 x 2.54 cm (1 x 1 inch) piece of polycaprolactone/polyglycolic acid (PCL/PGA) 90/10 copolymer film (at a density of about 86 g/m² (8 g/ft²)) was placed on top of a 2.54 x 2.54 cm (1 x 1 inch) piece of MARLEX® polypropylene mesh.

A HA/CMC composition was produced generally following the procedures described in U.S. Patent No. 5,017,229. A carbodiimide modified HA/CMC powder was suspended in deionized H₂O at a concentration of 1% (w/v) using a high shear blender (Turrax T50 with G45F head) for 10 minutes. The HA/CMC suspension was poured into a polystyrene tray or TEFLON®-coated stainless steel tray at a density of 43 g/m² (4 g/ft²) and lyophilized into solid foam sheets. The shelf temperature was ramped down to -20°C at a rate of 0.1 °C/min. The drying cycle was initiated with vacuum set at less than or equal to 20Pa (150 mtorr), and the shelf temperature was raised at 0.1 °C/min to -12°C. The shelf temperature was held at -12°C for 180 minutes and then raised at 0.1°C/min to 0°C. The shelf temperature was then held at 0°C for 900 minutes and then raised at 0.1°C/min to 27°C. The foam sheet was removed after the foam temperature reached the shelf temperature. Scanning electron microscopy of the resulting foam indicated a range of pore sizes about 50-300 µm in diameter.

A 2.54 x 2.54 cm (1 x 1 inch) piece of HA/CMC foam was placed on top of the PCUPGA film, which was on top of the polypropylene mesh. This configuration was placed between two TELFON®-coated stainless steel plates and pressed in a Carver Laboratory Press. The conditions for lamination were 121°C (250°F) for 30 seconds with no pressure, then 15 seconds at 2 metric tons of pressure. The prosthesis was removed from the press, allowed to cool and then removed from between the TELFON®-coated stainless steel plates. The HA/CMC component of the one piece prosthesis did not exhibit any discoloration, nor did the polypropylene show signs of melting. In addition, the various layers of the prosthesis were well-incorporated into adjacent layers.

### Example 2

A 5.08 x 5.08 cm (2 x 2 inch) piece of PCUPGA 95/5 copolymer film (at a density of 86 g/m² (8 g/ft²)) was placed on top of a 5.08 x 5.08 cm (2 x 2 inch) piece of MARLEX® polypropylene mesh. Then a 5.08 x 5.08 cm (2 x 2 inch) piece of HA/CMC foam, produced according to Example 1, was placed on top of the PCL/PGA film. This sandwich was placed between two TEFLON® sheets with a 0.4 mm spacer and then pressed between chrome plates using a Carver Laboratory Press. The spacer is a metal shim placed between the chrome plates that allows for the maintenance of a predetermined gap thickness and prevents pressing the mesh completely through the surface of the foam. The conditions for lamination were 104°C (220°F) for 45 seconds with no pressure, then 15 seconds at 2 metric tons pressure. The material was removed from the press, allowed to cool at 4°C, and then removed from the plates. The PCL/PGA, HA/CMC foam, and mesh components were all well-incorporated into adjacent layers in the prosthesis.

To determine whether the adhesive was necessary to produce a well-adhered, uniform distribution of HA/CMC over the polypropylene mesh, a 3.18 x 10.48 cm (1.25 x 4.125 inch) piece of mesh was placed in a TEFLON®-coated tray. A 0.6% (w/v) suspension of HA/CMC was poured over the mesh to achieve a density of 48 g/m² (4.5 g/ft²) and lyophilized into a solid foam sheet as described in Example 1. The distribution of the HA/CMC foam matrix on the polypropylene mesh was uneven, with regions of the mesh having dense coverage of HA/CMC and other regions of the mesh having light coverage of HA/CMC. Thus, the adhesive was desirable for producing a prosthesis having an even distribution of HA/CMC over a mesh.

The role of the adhesive in the preparation of the prosthesis was further investigated by eliminating the adhesive in another method for producing a prosthesis. A 2.54 x 2.54 cm (1 x 1 inch) piece of HA/CMC foam produced as described in Example 1 was placed on top of a 2.54 x 2.54 cm (1 x 1 inch) piece of MARLEX® polypropylene mesh, then pressed between two TEFLON®-coated stainless steel plates using a Carver Laboratory Press. The foam and mesh were then laminated by heating at 166-177°C (330-350°F) for 30 seconds with no pressure, then for 15 seconds at 2 metric tons of pressure. The prosthesis was removed from the press, allowed to cool, and removed from the stainless steel plates. There was poor or no incorporation of the HA/CMC foam into the mesh. Thus, the adhesive was desirable for incorporation of the layers of the prosthesis into adjacent layers. In addition, it was concluded that the relatively neutral (in terms of hydrophobicity) adhesive facilitated contact between two dislike materials: a hydrophobic non-bioabsorbable synthetic polymer mesh and a hydrophilic bioabsorbable polymer.

### Example 3

HA/CMC foam produced according to Example I was subjected to dehydrotherrnal treatment (DHT) at 100°C for 6 hours. A 5.72 x 5.72 cm (2.25 x 2.25 inch) piece of polyglycolic acid/poiytactic acid (PGA/PLA) 50/50 copolymer film (at a density of about 65 g/m² (6g/ft²)) was placed on top of a 5.72 x 5.72 cm (2.25 x 2.25 inch) piece of the foam after DHT. Then the 5.72 x 5.72 cm (2.25. x 2.25 inch) piece of MARLEX® polypropylene mesh was placed on top of the PGA/PLA film. This configuration was placed between two TEFLON® sheets with a 0.4 mm spacer and pressed between two chrome plates using a Carver Laboratory Press. The conditions for lamination were 116°C (240°F) for 45 seconds with no pressure, then pressing for 20 seconds at 1362 kg (3000 pounds (lb)) pressure. The material was removed from the press, allowed to cool at 4°C, and then removed from the plates. The PGA/PLA, HA/CMC foam, and mesh components were all well-incorporated into adjacent layers in the prosthesis.

### Example 4

Two 7.62 x 7.62 cm (3 x 3 inch) pieces of HA/CMC foam made according to Example 1 and two 7.62 x 7.62 cm (3 x 3 inch) pieces of MARLEX® polypropylene mesh were each spray-coated on one surface with a 3% (w/v) solution of PGA/PLA 50/50 copolymer in methylene chloride to achieve a 15% final weight gain. All four pieces were individually packaged in Chex-All® II pouches and subjected to (DHT) at 100°C for 6 hours. Then a single foam piece was placed on top of a single MARLEX® piece, with the spray-coated surface of one piece contacting the spray-coated surface of the other piece. This configuration was placed between two TEFLON® sheets with a 1.45 mm spacer and pressed between two chrome plates using a Carver Laboratory Press. The conditions for lamination were 116°C (240°F) for 1 minute with no pressure, then 30 seconds at 3178 kg (7000 lb) pressure. The material was removed from the press, allowed to cool at 4°C, and then removed from the plates. The PGA/PLA, HA/CMC foam, and mesh components were all well-incorporated into adjacent layers in the prosthesis. This procedure was repeated for the remaining two pieces.

To determine the desirability of the porous nature of the HA/CMC foam in the prostheses produced immediately above, a 5.08 x 5.08 cm (2 x 2 inch) piece of non-porous, glycerol plasticized HA/CMC film and a 5.08 x 5.08 cm (2 x 2 inch) piece of BARD® mesh (Davol, Inc.) were spray-coated on a surface with a 3% (w/v) solution of PGA/PLA 50/50 copolymer in methylene chloride to achieve a 15% final polymer weight gain for each of the film and mesh. The non-porous HA/CMC film was placed on top of the mesh with the spray-coated surfaces contacting each other. This configuration was placed between two TEFLON® sheets with a 0.625 mm spacer and then pressed between two chrome plates using a Carver Laboratory Press. The film and mesh were then laminated at 116°C (240°F) for 1 minute with no pressure, then for 45 seconds at 454 kg (1000 lb) pressure. The prosthesis was removed from the press, allowed to cool in a 2-8°C cold room, and removed from between the chrome plates. The prosthesis exhibited little, if any, infiltration of the mesh by the film, in contrast to the prosthesis having the porous HA/CMC foam.

The practical effect of the minimal infiltration in the nonporous HA/CMC film prosthesis was also compared to the porous HA/CMC foam prosthesis. Both types of prostheses produced in this Example were evaluated for surgical handling and placement properties in a rabbit hernia repair model (Dinsmore, The American Surgeon 65:383-387, 1999). When the prosthesis having the porous HA/CMC foam layer was prehydrated prior to the surgical insertion, the prosthesis did not delaminate. The prosthesis could be handled adequately to surgically repair the defect in the animal. In contrast, the prosthesis having the nonporous HA/CMC film layer delaminated into two pieces when prehydrated and was not able to be used to repair the body cavity opening in the rabbit hernia repair model.

To evaluate the physical strength and integrity of the porous HA/CMC foam prosthesis versus the nonporous HA/CMC film prosthesis, the wet bond strength of each was determined using an in vitro phosphate buffered saline (PSB) immersion assay. 2.54 x 2.54 cm (1 x 1 inch) sample prostheses were each placed into a 50 ml vial filled with 30 ml of PBS. The vials were then moderately agitated at 37°C. The samples were then removed from the vials, placed in 100% ethanol to remove the water, and then stained with the dye alcian blue, which binds to HA. The extent of staining correlated with the amount of HA remaining on a mesh. The results from the PBS immersion assay are summarized in Table 1.

**TABLE 1**

| Duration of Immersion | | |
|---|---|---|
| Test Group | | Assessment |
| Foam Prosthesis | 2 hours | HA present; 100% of mesh covered with dark blue staining |
| | | |
| Film Prosthesis | 2 hours | no HA present; no staining |
| | | |
| Foam Prosthesis | 6 hours | HA present, 100% of mesh covered with dark blue staining |
| | | |
| Film Prosthesis | 6 hours | no HA present, no staining |

The lack of staining for the nonporous film prosthesis appeared to be due to delamination of the film from the mesh after 2 or more hours of immersion. In contrast, the porous foam prosthesis retained HA after 6 hours of immersion.

### Example 5

The prosthesis having a porous HA/CMC foam layer prepared in Example 4 was further evaluated in a rat hernia repair model (Dinsmore, supra) and was compared with the performance of MARLEX® polypropylene mesh only. A 2.54 x 2.54 cm (1 x 1 inch) full thickness defect was excised from the rectus abdominis muscle of rats to prepare the animal model. The defect was repaired by suturing the test material into the defect using a continuous suture pattern. In one set of animals, the composite prosthesis was used for repair of the hernia, with the HA/CMC layer facing the viscera. In another set of animals, MARLEX® mesh only was used for repair of the hernia. The rats were sacrificed 28 days after repair and graded for adhesion formation using the following scale: 0 = no adhesions, 1 = less than or equal to 25% of the defect covered by adhesions, 2 = 26 to 50% of the defect covered by adhesions, 3 = 51 to 75% of the defect covered by adhesions, and 4 = greater than 75% of the defect covered by adhesions. The results are shown in Table 2.

**TABLE 2**

| Test Group | Average Extent of Adhesions | % of Animals w/no Adhesions |
|---|---|---|
| Polypropylene Mesh Only (n=5) | 3.0 ± 1.0 | 0 |
| Composite Prosthesis (n=10) | 0.7 ± 0.7* | 40 |

| | | |
|---|---|---|
| *Significantly different from mesh only group (Tukey-Kramer, p ≤ 0.05) Average extent of adhesions is expressed as the mean ± one standard deviation. | | |

The results in Table 2 indicated that the composite prosthesis of the invention was superior to the polypropylene mesh in preventing adhesions in vivo.

Other embodiments are included within the following claims. For example, the bioabsorbable layer may contain chitosan, alginate, or other bioabsorbable materials or combinations of materials. In addition, the prosthesis may contain a protein drug, non-steroidal anti-inflammatory drug, small molecule drug, or the like. The drug may be incorporated in any portion of the prosthesis (e.g., the bioabsorbable layer or the adhesive) to provide for the controlled release of the drug into the body cavity to be repaired with the prosthesis. Similarly, the mesh layer may be formed of a variety of materials that are not reactive or minimally reactive with the tissue of the patient.

## Claims

1. A surgical prosthesis comprising:
a non-bioabsorbable layer having an outer wall-facing surface and an inner bioabsorbable layer-facing surface;
an adhesive; and
a bioabsorbable layer comprising a material selected from the group consisting of hyaluronic acid and carboxymethyl cellulose, the bioabsorbable layer having an outer viscera-facing surface and an inner non-bioabsorbable layer-facing surface attached to the bioabsorbable layer-facing surface of the non-bioabsorbable layer by the adhesive.

2. The prosthesis of Claim 1 wherein the bioabsorbable layer contains pores in the non-bioabsorbable layer-facing surface.

3. The prosthesis of Claim 2 wherein the pores traverse the bioabsorbable layer from the viscera-facing surface to the non-bioabsorbable layer-facing surface.

4. The prosthesis of Claim 2 or Claim 3 wherein the pores are about 10-500µm, preferably about 30-300µm, more preferably about 40-100µm in diameter.

5. The prosthesis of any preceding claim wherein the bioabsorbable layer comprises hyaluronic acid and carboxymethyl cellulose.

6. The prosthesis of Claim 5 wherein the ratio of the total amount of hyaluronic acid to the total amount of carboxymethylcellulose in the bioabsorbable layer is about 2:1.

7. The prosthesis of any preceding claim wherein the non-bioabsorbable layer comprises polypropylene or poly(ethylene terephthalate).

8. The prosthesis of any preceding claim wherein the non-bioabsorbable layer is a mesh.

9. The prosthesis of any preceding claim wherein the adhesive is bioabsorbable.

10. The prosthesis of any preceding claim wherein the adhesive comprises polyglycolic acid, polylactic acid, polycaprolactone, polydioxanone, polyestercarbonate, polyhydroxyalkonate or copolymers thereof.

11. The prosthesis of Claim 10 wherein the adhesive comprises a copolymer of polylactic acid and polyglycolic acid, and the ratio of the total amount of polyglycolic acid to the total amount of polylactic acid in the adhesive is about 1:1.

12. The prosthesis of any preceding claim wherein the non-bioabsorbable layer has a density of about 68 g/m² (6.3 g/ft²), the bioabsorbable layer has a density of about 22 to 48 g/m² (2.0 to 4.5 g/ft²), and the adhesive has a density of about 29 to 44 g/m² (2.7 to 4.1 g/ft²).

13. A surgical prosthesis according to any preceding claim for use in repairing an opening in a wall enclosing a body cavity of a human patient.

14. A method of producing a prosthesis, the method comprising providing a non-bioabsorbable sheet;
applying an adhesive to a surface of the non-bioabsorbable sheet; and
adhering a bioabsorbable composition to the surface subsequent to the application of the adhesive to the surface, the bioabsorbable composition comprising a material selected from the group consisting of hyaluronic acid and carboxymethylcellulose.

15. The method of Claim 14 wherein the adhering step comprises placing the bioabsorbable layer onto the surface and heat-pressing the composition to the surface.

## Patentansprüche

1. Eine chirurgische Prothese mit:
einer biologisch nicht absorbierbaren Schicht, die eine der Wand zugewandten Außenfläche und eine der biologisch absorbierbaren Schicht zugewandten Innenfläche hat;
einem Klebstoff; und
einer biologisch absorbierbaren Schicht, die ein Material enthält, das aus der aus Hyaluronsäure und Carboxymethylzellulose bestehenden Gruppe ausgewählt wird, wobei die biologisch absorbierbare Schicht eine den Eingeweiden zugewandte Außenfläche und eine der nicht biologisch absorbierbaren Schicht zugewandte Innenfläche aufweist, die mittels des Klebstoffes mit derjenigen Seite der biologisch nicht absorbierbaren Schicht verbunden ist, die der biologisch absorbierbaren Schicht zugewandt ist.

2. Die Prothese nach Anspruch 1, bei der die biologisch absorbierbare Schicht auf der Seite, die der biologisch nicht absorbierbaren Schicht zugewandt ist, Poren aufweist.

3. Die Prothese nach Anspruch 2, bei der sich die Poren in der biologisch absorbierbaren Schicht von der den Eingeweiden zugewandten Seite bis zu der Seite erstrecken, die der biologisch nicht absorbierbaren Schicht zugewandt ist.

4. Die Prothese nach Anspruch 2 oder Anspruch 3, bei der die Poren einen Durchmesser von etwa 10 bis 500 µm, vorzugsweise etwa 30 bis 300 µm und noch besser etwa 40 bis 100 µm haben.

5. Die Prothese nach einem der vorangehenden Ansprüche, bei der die biologisch absorbierbare Schicht Hyaluronsäure und Carboxymethylzellulose enthält.

6. Die Prothese nach Anspruch 5, bei der das Verhältnis von Gesamt-Hyaluronsäure zu Gesamt-Carboxymethylzellulose in der biologisch absorbierbaren Schicht ungefähr 2:1 beträgt.

7. Die Prothese eines der vorstehenden Ansprüche, bei der die biologisch nicht absorbierbare Schicht Polypropylen oder Poly(ethylenterephthalat) enthält.

8. Die Prothese eines der vorstehenden Ansprüche, bei der die biologisch nicht absorbierbare Schicht aus einem Netz besteht.

9. Die Prothese eines der vorstehenden Ansprüche, bei der der Klebstoff biologisch absorbierbar ist.

10. Die Prothese eines der vorstehenden Ansprüche, bei der der Klebstoff Polyglycolsäure, Polymilchsäure, Polykaprolakton, Polydioxanon, Polyestercarbonat, Polyhydroxyalkonat oder Copolymere davon enthält.

11. Die Prothese nach Anspruch 10, bei der der Klebstoff ein Copolymer aus Polymilchsäure und Polyglycolsäure enthält und das Verhältnis von Gesamt-Polyglycolsäure zu Gesamt-Polymilchsäure im Klebstoff ungefähr 1:1 beträgt.

12. Die Prothese eines der vorstehenden Ansprüche, bei der die biologisch nicht absorbierbare Schicht eine Dichte von etwa 68 g/m², die biologisch absorbierbare Schicht eine Dichte von etwa 22 bis 48 g/m² und der Klebstoff eine Dichte von etwa 29 bis 44 g/m² hat.

13. Eine chirurgische Prothese nach einem der vorstehenden Ansprüche, die dazu verwendet wird, eine Öffnung in einer eine Körperhöhle eines menschlichen Patienten umschließenden Wand zu reparieren.

14. Ein Verfahren zur Herstellung einer Prothese, wobei das Verfahren die Erstellung einer biologisch nicht absorbierbaren Folie;
das Aufstreichen eines Klebstoffes auf eine Oberfläche der biologisch nicht absorbierbaren Folie; und
nach dem Aufstreichen des Klebstoffs auf die Oberfläche das Aufbringen einer biologisch absorbierbaren Verbindung auf diese Oberfläche beinhaltet und wobei die biologisch absorbierbare Verbindung ein Material enthält, das aus der aus Hyaluronsäure und Carboxymethylzellulose bestehenden Gruppe ausgewählt wird.

15. Das Verfahren nach Anspruch 14, bei dem der Vorgang des Aufbringens darin besteht, die biologisch absorbierbare Schicht auf die Oberfläche aufzulegen und die Verbindung unter Hitzeeinwirkung auf die Oberfläche zu pressen.

## Revendications

1. Prothèse chirurgicale comprenant :
une couche non bioabsorbable possédant une surface extérieure faisant face à la paroi et une surface intérieure faisant face à la couche bioabsorbable ;
un adhésif ; et
une couche bioabsorbable comprenant un matériau choisi dans le groupe constitué par un acide hyaluronique et une carboxyméthylcellulose, la couche bioabsorbable possédant une surface extérieure faisant face aux viscères et une surface intérieure faisant face à la couche non bioabsorbable reliée à la surface de la couche non bioabsorbable faisant face à la couche bioabsorbable, au moyen de l'adhésif.

2. Prothèse selon la revendication 1, dans laquelle la couche bioabsorbable contient des pores dans la surface faisant face à la couche non bioabsorbable.

3. Prothèse selon la revendication 2, dans laquelle les pores traversent la couche bioabsorbable depuis la surface faisant face aux viscères jusqu'à la surface faisant face à la couche non bioabsorbable.

4. Prothèse selon la revendication 2 ou la revendication 3, dans laquelle les pores mesurent environ 10 à 500 µm, de préférence environ 30 à 300 µm, plus préférentiellement environ 40 à 100 µm de diamètre.

5. Prothèse selon l'une quelconque des revendications précédentes, dans laquelle la couche bioabsorbable comprend de l'acide hyaluronique et de la carboxyméthylcellulose.

6. Prothèse selon la revendication 5, dans laquelle le rapport de la quantité totale d'acide hyaluronique à la quantité totale de carboxyméthylcellulose dans la couche bioabsorbable est d'environ 2:1.

7. Prothèse selon l'une quelconque des revendications précédentes, dans laquelle la couche non bioabsorbable comprend du polypropylène ou du poly(téréphtalate d'éthylène).

8. Prothèse selon l'une quelconque des revendications précédentes, dans laquelle la couche non bioabsorbable est un maillage.

9. Prothèse selon l'une quelconque des revendications précédentes, dans laquelle l'adhésif est bioabsorbable.

10. Prothèse selon l'une quelconque des revendications précédentes, dans laquelle l'adhésif comprend de l'acide polyglycolique, de l'acide polylactique, une polycaprolactone, une polydioxanone, un polyester carbonate, un polyhydroxyalkonate ou des copolymères de ceux-ci.

11. Prothèse selon la revendication 10, dans laquelle l'adhésif comprend un copolymère d'acide polylactique et d'acide polyglycolique, et le rapport de la quantité totale d'acide polyglycolique à la quantité totale d'acide polylactique est d'environ 1:1.

12. Prothèse selon l'une quelconque des revendications précédentes, dans laquelle la couche non bioabsorbable possède une densité d'environ 68 g/m² (6,3 g/pied²), la couche bioabsorbable possède une densité d'environ 22 à 48 g/m² (2 à 4,5 g/pied²), et l'adhésif possède une densité d'environ 29 à 44 g/m² (2,7 à 4,1 g/pied²).

13. Prothèse chirurgicale selon l'une quelconque des revendications précédentes pour utilisation dans la réparation d'une ouverture dans une paroi renfermant une cavité corporelle d'un patient humain.

14. Procédé de production d'une prothèse, le procédé comprenant la fourniture d'une feuille non bioabsorbable ;
l'application d'un adhésif sur une surface de la feuille non bioabsorbable ; et
l'adhérence d'une composition bioabsorbable sur la surface à la suite de l'application de l'adhésif sur la surface, la composition bioabsorbable comprenant un matériau choisi dans le groupe constitué par un acide hyaluronique et une carboxyméthylcellulose.

15. Procédé selon la revendication 14, dans lequel l'étape d'adhérence comprend le placement de la couche bioabsorbable sur la surface et le pressage à chaud de la composition sur la surface.
